# EUROPEAN PATENT APPLICATION

(11) **EP 4 272 759 A1**
(43) Date of publication of application: **08.11.2023**
(21) Application number: 22749846.6
(22) Date of filing: 07.02.2022
(51) Int. Cl.: A61K 45/00, A61K 38/06, A61P 29/00, A61P 43/00

(54) **PREVENTIVE OR THERAPEUTIC AGENT FOR INFLAMMATORY DISORDERS AND/OR PAIN DISORDERS**

(30) Priority: 08.02.2021 JP 2021018177
(71) Applicant: Kyowa Pharma Chemical Co., Ltd., Takaoka-shi, Toyama 933-8511 (JP)
(72) Inventor: OHSHIMA Etsuo, Takaoka-shi, Toyama 933-8511 (JP)
(74) Representative: Vossius & Partner Patentanwälte Rechtsanwälte mbB
(86) International application number: PCT/JP2022/004692
(87) International publication number: WO 2022/168975

(57) **Abstract**

The present invention exhibits strong preventive effects and/or therapeutic effects with regard to inflammatory disorders and/or pain disorders by combining and administering glutathione trisulfide or a pharmaceutically-acceptable salt thereof and a non-steroidal anti-inflammatory drug or a pharmaceutically acceptable salt thereof.

## Description

### Technical Field

The present invention relates to a preventive or therapeutic agent for an inflammatory disease and/or a pain disorder.

### Background Art

Non-steroidal anti-inflammatory drugs (NSAIDs) are drugs that exert anti-inflammatory, antipyretic, analgesic, and anticoagulant effects by inhibiting cyclooxygenase (COX) which is a prostaglandin synthetase, and are widely used for medical treatment (for example, Patent Literature 1).

Polysulfides such as glutathione trisulfide (GSSSG) are converted *in vivo* to reactive sulfur species such as oxidized glutathione (GSSH). It has been reported that reactive sulfur species have a potent antioxidant effect and may have physiological functions such as antiaging (for example, Non Patent Literature 1 and 2).

### Citation List

### Patent Literature

[Patent Literature 1] JP 2021-500384

### Non Patent Literature

[Non-Patent Literature 1] T. Ida, et al., PNAS, May 27, 2014, 111 (21) 7606-7611
[Non-Patent Literature 2] T. Akaike, et al., Nature Communications, 2017, Oct 27; 8 (1): 1177

### Summary of Invention

### Technical Problem

An object of the present invention is to provide a preventive or therapeutic agent for an inflammatory disease and/or a pain disorder having a highly preventive or therapeutic effect.

### Solution to Problem

The present inventors have found that administration of a combination of a non-steroidal anti-inflammatory drug and glutathione trisulfide shows a highly preventive or therapeutic effect on an inflammatory disease and/or a pain disorder, thus leading to realization of the present invention.

The present invention provides [1] to [46] below.
[1] A preventive or therapeutic agent for an inflammatory disease and/or a pain disorder, comprising a non-steroidal anti-inflammatory drug or a pharmaceutically acceptable salt thereof, which is administered in combination with glutathione trisulfide or a pharmaceutically acceptable salt thereof.
[2] A preventive or therapeutic agent for an inflammatory disease and/or a pain disorder, comprising glutathione trisulfide or a pharmaceutically acceptable salt thereof, which is administered in combination with a non-steroidal anti-inflammatory drug or a pharmaceutically acceptable salt thereof.
[3] The preventive or therapeutic agent according to [1] or [2], wherein glutathione trisulfide or the pharmaceutically acceptable salt thereof and the non-steroidal anti-inflammatory drug or the pharmaceutically acceptable salt thereof are administered simultaneously or separately.
[4] A preventive or therapeutic agent for an inflammatory disease and/or a pain disorder, comprising glutathione trisulfide or a pharmaceutically acceptable salt thereof; and a non-steroidal anti-inflammatory drug or a pharmaceutically acceptable salt thereof.
[5] The preventive or therapeutic agent according to any one of [1] to [4], wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.
[6] The preventive or therapeutic agent according to any one of [1] to [4], wherein glutathione trisulfide or the pharmaceutically acceptable salt thereof contains at least one selected from the group consisting of glutathione trisulfide, an arginine salt of glutathione trisulfide, and a sodium salt of glutathione trisulfide.
[7] The preventive or therapeutic agent according to any one of [1] to [6], wherein the non-steroidal anti-inflammatory drug or a pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
[8] A kit for preventing or treating an inflammatory disease and/or a pain disorder, comprising a preparation comprising glutathione trisulfide or a pharmaceutically acceptable salt thereof; and a preparation comprising a non-steroidal anti-inflammatory drug or a pharmaceutically acceptable salt thereof.
[9] The kit according to [8], wherein the preparation comprising glutathione trisulfide or the pharmaceutically acceptable salt thereof and the preparation comprising the non-steroidal anti-inflammatory drug or the pharmaceutically acceptable salt thereof are administered simultaneously or separately.
[10] The kit according to [8] or [9], wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.
[11] The kit according to [8] or [9], wherein glutathione trisulfide or the pharmaceutically acceptable salt thereof contains at least one selected from the group consisting of glutathione trisulfide, an arginine salt of glutathione trisulfide, and a sodium salt of glutathione trisulfide.
[12] The kit according to any one of [8] to [11], wherein the non-steroidal anti-inflammatory drug or the pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
[13] A method for preventing or treating an inflammatory disease and/or a pain disorder, comprising administering glutathione trisulfide or a pharmaceutically acceptable salt thereof and a non-steroidal anti-inflammatory drug or a pharmaceutically acceptable salt thereof to a patient in need thereof.
[14] The method according to [13], wherein glutathione trisulfide or the pharmaceutically acceptable salt thereof and the non-steroidal anti-inflammatory drug or the pharmaceutically acceptable salt thereof are administered simultaneously or separately.
[15] The method according to [13] or [14], wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.
[16] The method according to [13] or [14], wherein glutathione trisulfide or a pharmaceutically acceptable salt thereof contains at least one selected from the group consisting of glutathione trisulfide, an arginine salt of glutathione trisulfide, and a sodium salt of glutathione trisulfide.
[17] The method according to any one of [13] to [16], wherein the non-steroidal anti-inflammatory drug or a pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
[18] A non-steroidal anti-inflammatory drug or a pharmaceutically acceptable salt thereof for use in prevention or treatment of an inflammatory disease and/or a pain disorder, which is administered in combination with glutathione trisulfide or a pharmaceutically acceptable salt thereof.
[19] The non-steroidal anti-inflammatory drug or the pharmaceutically acceptable salt thereof for use according to [18], which is administered simultaneously with or separately from glutathione trisulfide or the pharmaceutically acceptable salt thereof.
[20] The non-steroidal anti-inflammatory drug or the pharmaceutically acceptable salt thereof for use according to [18] or [19], wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.
[21] The non-steroidal anti-inflammatory drug or the pharmaceutically acceptable salt thereof for use according to [18] or [19], wherein glutathione trisulfide or the pharmaceutically acceptable salt thereof contains at least one selected from the group consisting of glutathione trisulfide, an arginine salt of glutathione trisulfide, and a sodium salt of glutathione trisulfide.
[22] The non-steroidal anti-inflammatory drug or the pharmaceutically acceptable salt thereof for use according to any one of [18] to [21], which contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
[23] Glutathione trisulfide or a pharmaceutically acceptable salt thereof for use in prevention or treatment of an inflammatory disease and/or a pain disorder, which is administered in combination with a non-steroidal anti-inflammatory drug or a pharmaceutically acceptable salt thereof.
[24] Glutathione trisulfide or the pharmaceutically acceptable salt thereof for use according to [23], which is administered simultaneously with or separately from the non-steroidal anti-inflammatory drug or the pharmaceutically acceptable salt thereof.
[25] Glutathione trisulfide or the pharmaceutically acceptable salt thereof for use according to [23] or [24], which contains at least one selected from the group consisting of glutathione trisulfide, an amino acid salt of glutathione trisulfide, and an alkali metal salt of glutathione trisulfide.
[26] Glutathione trisulfide or the pharmaceutically acceptable salt thereof for use according to [23] or [24], which contains at least one selected from the group consisting of glutathione trisulfide, an arginine salt of glutathione trisulfide, and a sodium salt of glutathione trisulfide.
[27] Glutathione trisulfide or a pharmaceutically acceptable salt thereof for use according to any one of [23] to [26], wherein the non-steroidal anti-inflammatory drug or the pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
[28] A combination of a non-steroidal anti-inflammatory drug or a pharmaceutically acceptable salt thereof and glutathione trisulfide or a pharmaceutically acceptable salt thereof for use in prevention or treatment of an inflammatory disease and/or a pain disorder.
[29] The combination for use according to [28], wherein glutathione trisulfide or the pharmaceutically acceptable salt thereof and the non-steroidal anti-inflammatory drug or the pharmaceutically acceptable salt thereof are administered simultaneously or separately.
[30] The combination for use according to [28] or [29], wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.
[31] The combination for use according to [28] or [29], wherein glutathione trisulfide or the pharmaceutically acceptable salt thereof contains at least one selected from the group consisting of glutathione trisulfide, an arginine salt of glutathione trisulfide, and a sodium salt of glutathione trisulfide.
[32] The combination for use according to any one of [28] to [31] wherein the non-steroidal anti-inflammatory drug or a pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
[33] Use of a non-steroidal anti-inflammatory drug or a pharmaceutically acceptable salt thereof for the manufacture of a preventive or therapeutic agent for an inflammatory disease and/or a pain disorder, which is administered in combination with glutathione trisulfide or a pharmaceutically acceptable salt thereof.
[34] The use according to [33], wherein glutathione trisulfide or the pharmaceutically acceptable salt thereof and the non-steroidal anti-inflammatory drug or a pharmaceutically acceptable salt thereof are administered simultaneously or separately.
[35] The use according to [33] or [34], wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.
[36] The use according to [33] or [34], wherein glutathione trisulfide or the pharmaceutically acceptable salt thereof contains at least one selected from the group consisting of glutathione trisulfide, an arginine salt of glutathione trisulfide, and a sodium salt of glutathione trisulfide.
[37] The use according to any one of [33] to [36], wherein the non-steroidal anti-inflammatory drug or the pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
[38] Use of glutathione trisulfide or a pharmaceutically acceptable salt thereof for the manufacture of a preventive or therapeutic agent for an inflammatory disease and/or a pain disorder, which is administered in combination with non-steroidal anti-inflammatory drug or a pharmaceutically acceptable salt thereof.
[39] The use according to [38], wherein glutathione trisulfide or the pharmaceutically acceptable salt thereof and the non-steroidal anti-inflammatory drug or the pharmaceutically acceptable salt thereof are administered simultaneously or separately.
[40] The use according to [38] or [39], wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.
[41] The use according to [38] or [39], wherein glutathione trisulfide or the pharmaceutically acceptable salt thereof contains at least one selected from the group consisting of glutathione trisulfide, an arginine salt of glutathione trisulfide, and a sodium salt of glutathione trisulfide.
[42] The use according to any one of [38] to [41], wherein the non-steroidal anti-inflammatory drug or a pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
[43] Use of a combination of glutathione trisulfide or a pharmaceutically acceptable salt thereof and a non-steroidal anti-inflammatory drug or a pharmaceutically acceptable salt thereof for the manufacture of a preventive or therapeutic agent for an inflammatory disease and/or a pain disorder.
[44] The use according to [43], wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.
[45] The use according to [43], wherein glutathione trisulfide or the pharmaceutically acceptable salt thereof contains at least one selected from the group consisting of glutathione trisulfide, an arginine salt of glutathione trisulfide, and a sodium salt of glutathione trisulfide.
[46] The use according to any one of [43] to [45], wherein the non-steroidal anti-inflammatory drug or a pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.

### Advantageous Effects of Invention

According to the present invention, it is possible to provide a preventive or therapeutic agent for an inflammatory disease and/or a pain disorder having a highly preventive or therapeutic effect.

### Brief Description of Drawings

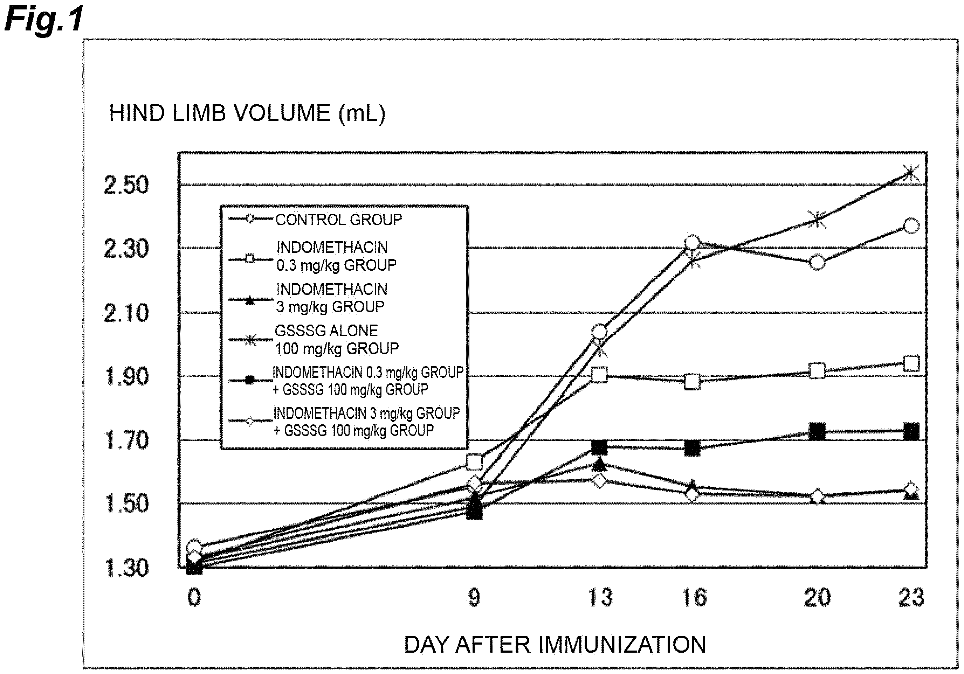

FIG. 1 is a graph showing changes in hind limb volume in adjuvant arthritis model rats in cases where indomethacin and glutathione trisulfide are administered alone and in combination.

### Description of Embodiments

A preventive or therapeutic agent and a preventive or therapeutic method of the present invention may be administered or applied to humans.

An inflammatory disease in the present invention refer to a disease in which inflammation is one of the etiologies. The preventive or therapeutic agent and the preventive or therapeutic method of the present invention are thought to have a highly preventive or therapeutic effect not only for inflammatory diseases but also for pain disorders in which pain is one of the symptoms and for thrombotic diseases in which formation of thrombi is one of the etiologies. Examples of these diseases including inflammatory diseases include systemic lupus erythematosus, scleroderma, atopic dermatitis, contact dermatitis, psoriasis, rheumatoid arthritis, osteoarthritis, interstitial pneumonia, bronchial asthma, upper respiratory inflammation, tonsillitis, chronic obstructive pulmonary disease, pulmonary hypertension, inflammatory bowel disease (IBD) such as ulcerative colitis and Crohn's disease, nerve injury, spinal cord injury, spinal canal stenosis, stroke (sequelae of cerebral infarction or hemorrhage), amyotrophic lateral sclerosis, chronic inflammatory demyelinating polyneuropathy, multiple sclerosis, Parkinson's disease, dementia such as Alzheimer's disease and vascular dementia, schizophrenia, rejection during organ transplantation, acute kidney injury, diabetic kidney failure, lupus nephritis, pyelonephritis, IgA nephropathy, chronic kidney disease, neuropathic pain, pain due to osteoporosis, pain due to cancer, pain after surgery or trauma, pain due to administration of anticancer agents, pain due to shingles, dental pain, migraine, myalgia, backache, arthralgia, menstrual pain, dysmenorrhea, burns, transient ischemic attack seizures, myocardial infarction, atrial fibrillation, fibromyalgia, vascular endothelialitis, Kawasaki disease, sequelae of viral infections, thrombosis due to artificial heart valves, thrombosis after surgery, and prostaglandin-involving cancers such as colon cancer, rectal cancer, and esophageal cancer.

In the present invention, examples of pharmaceutically acceptable salts include: salts with inorganic acids such as hydrochloric acid, hydrobromic acid, sulfuric acid, nitric acid, and phosphoric acid; salts with organic acids such as acetic acid, succinic acid, fumaric acid, maleic acid, tartaric acid, citric acid, lactic acid, stearic acid, benzoic acid, methanesulfonic acid, ethanesulfonic acid, and p-toluenesulfonic acid; salts with alkali metals such as sodium and potassium; salts with alkaline earth metals such as calcium and magnesium; ammonium salts; and salts with amino acids such as arginine.

Examples of non-steroidal anti-inflammatory drugs (NSAIDs) include NSAIDs based on: salicylic acid such as acetylsalicylic acid; propionic acid such as ibuprofen, loxoprofen, naproxen, ketoprofen, and flurbiprofen; acetic acid such as indomethacin and diclofenac; coxibs such as celecoxib and rofecoxib; and aniline such as acetaminophen, mefenamic acid, and tolfenamic acid.

Glutathione trisulfide (GSSSG) can increase glutathione levels in a living organism, and it can efficiently protect cells from peroxide or active oxygen species when the glutathione levels in a living organism increase because glutathione has a potent antioxidant effect.

In the present invention, the term "administration in combination" or "combined use" means use of a combination of active components and includes (1) a mode of administering a single preparation comprising NSAIDs and GSSSG and (2) a mode of administering NSAIDs and GSSSG as separate preparations simultaneously or separately with a time lag. In the case of (2), NSAIDs may be administered first, or GSSSG may be administered first. In addition, in the case of (2), either (i) a mode in which NSAIDs and GSSSG are formulated separately and administered simultaneously via the same route of administration, (ii) a mode in which NSAIDs and GSSSG are formulated separately and administered separately with a time lag via the same route of administration, (iii) a mode in which NSAIDs and GSSSG are formulated separately and administered simultaneously via different routes of administration (administration to different sites in the same patient), or (iv) a mode in which NSAIDs and GSSSG are formulated separately and administered separately through different routes of administration with a time lag may be adopted. In the case of (i), both drugs may be mixed immediately before administration.

In other words, the term "administration in combination" or "combined use" in the present invention can also be referred to as a mode in which either drug is administered and used in a state where actions and effects (such as an anti-inflammatory action, an analgesic action, and an anticoagulant action) of the other drug is exhibited in the body of a patient. That is, in the present invention, a mode in which NSAIDs and GSSSG are administered so as to be simultaneously present in the body of a patient, for example, in blood is preferable, and a mode in which one drug is administered to a patient within 24 hours of administering the other drug is preferable.

The dose of NSAIDs per kilogram of body weight is preferably 0.1 to 50 mg per day (0.1 to 50 mg/kg/day), more preferably 0.5 to 20 mg/kg/day, and still more preferably 1 to 10 mg/kg/day is still more preferable.

The dose of GSSSG per kilogram of body weight is preferably 0.5 to 1,000 mg per day (0.5 to 1,000 mg/kg/day), more preferably 1 to 400 mg/kg/day, and still more preferably 2 to 200 mg/kg/day.

It is preferable that the doses of NSAIDs and GSSSG be respectively 0.1 to 50 mg/kg/day and 0.5 to 1,000 mg/kg/day, it is more preferable that the doses of NSAIDs and GSSSG be respectively 0.5 to 20 mg/kg/day and 1 to 400 mg/kg/day, and it is still more preferable that the doses of NSAIDs and GSSSG be respectively 1 to 10 mg/kg/day and 2 to 200 mg/kg/day. When the doses of NSAIDs and GSSSG are within these ranges, it is thought that a higher preventive effect for an inflammatory disease and/or a pain disorder is exhibited and a higher therapeutic effect is exhibited. In addition, an effect of further reducing side effects is also expected.

The number of times of administration of NSAIDs and GSSSG can be set to 1 to 6 times a day or 1 to 4 times a day. Such frequency of administration is considered to reduce the patient's burden of taking the drugs and improve drug compliance. As a result, it is expected that the side effect reducing effects, anti-inflammatory effects, and the like exhibited by the preventive or therapeutic agent of the present invention will be further improved. In a case where drug administration is discontinued, the dose of the drug may be reduced stepwise. For example, there is a method for administering one drug during a withdrawal period of the other drug.

The preventive or therapeutic agent for an inflammatory disease and/or a pain disorder of the present invention can be administered orally in the forms of solid preparations such as tablets, granules, fine granules, powders, and capsules, liquids, jelly, syrups, and the like. In addition, the preventive or therapeutic agent for an inflammatory disease and/or a pain disorder of the present invention may be administered parenterally in the forms of injections, suppositories, ointments, poultices, and sprays. These dosage forms can be formulated in accordance with well-known methods.

The preventive or therapeutic agent for an inflammatory disease and/or a pain disorder of the present invention may be obtained by mixing either or both of a first component and a second component with water, ethanol, physiological saline, liquid paraffin, and the like.

### [Examples]

Hereinafter, the present invention will be described in more detail with reference to examples. However, the present invention is not limited by these examples.

### <Synergistic effect of combined use in rat adjuvant arthritis models>

Required amounts of GSSSG and indomethacin were weighed with an electronic balance, each ground well with a mortar, added to a solvent (0.5% methyl cellulose (MC) suspension), and suspended. The administration solution of GSSSG alone was prepared to have a concentration of 21.2 mg/mL (20 mg/mL on an anhydrous basis), and the administration solution of indomethacin alone was prepared to have a concentration of 0.06 mg/mL or 0.6 mg/mL. A combined administration solution was prepared by mixing a suspension prepared with GSSSG at 42.4 mg/mL (40 mg/mL on an anhydrous basis) with 0.12 mg/mL and 1.2 mg/mL indomethacin suspensions in equal amounts. The administration solution was prepared for 2 weeks at a time and stored under refrigeration and light shielding conditions until administration.

An appropriate amount of killed *Mycobacterium tuberculosis* (M. Tuberculosis H37Ra; BD) was ground in a mortar, and liquid paraffin was added thereto to prepare an adjuvant to 5 mg/mL. As experimental animals, 6-week-old male LEW rats were used. General condition observation and weight measurement were carried out upon arrival, and the presence or absence of abnormalities was confirmed. The acclimation period was set to 7 days, and the general condition was observed once a day. On the last day of the acclimation period, left hind limb volume and body weight were measured to confirm that there were no abnormalities in body weight and general condition, and the animals were then used for a test.

On the last day of the acclimation period, the rats were sensitized with 50 µL of the prepared adjuvant under isoflurane inhalation anesthesia under the skin of the right hind limb plantar. On day 9 after the sensitization of the adjuvant, the rats were grouped as shown in Table 1 below. In grouping, the rats were assigned to 6 groups (n=5) to ensure equal left hind limb volume and body weight.

**[Table 1]**

| Group | Group name | Administration substance | Dose (mg/kg) | Number of animals (individual number) |
|---|---|---|---|---|
| 1 | Control | 0.5% MC | 0 | 5 (101-105) |
| 2 | Indomethacin alone | Indomethacin | 0.3 | 5 (201-205) |
| 3 | Indomethacin alone | Indomethacin | 3 | 5 (301-305) |
| 4 | GSSSG alone | GSSSG | 100 | 5 (401-405) |
| 5 | Combination of indomethacin + GSSSG | Indomethacin + GSSSG | 0.3 + 100 | 5 (501-505) |
| 6 | Combination of indomethacin + GSSSG | Indomethacin + GSSSG | 3 + 100 | 5 (601-605) |

The administration solution of indomethacin alone, the administration solution of GSSSG alone, and the combined administration solution were forcibly orally administered at a volume of 5 mL/kg for 14 days from the day of grouping. The dose was calculated based on the most recent body weight. The volume of the left hind limb (non-sensitized limb) was measured using a rat hind limb plantar edema volume measurement device (TK-101P; Natsume Seisakusho Co., Ltd.) on the last day of the acclimation period, the day of grouping, and twice a week after the day of grouping. The measurement was performed 5 times per rat per day, and an average value of 3 times excluding the highest and lowest values was taken as the volume. The results are shown in Table 2 and FIG. 1.

As shown in Table 2 and FIG. 1, although no anti-inflammatory effect was observed in the GSSSG alone administration group, a synergistic anti-inflammatory effect was observed in the administration group of a combination of GSSSG and indomethacin (0.3 mg/kg). In addition, it is thought that the degree of pain caused by inflammation is also improved along with such expression of the high anti-inflammatory effect.

**[Table 2]**

| | | Test period | | | | | |
|---|---|---|---|---|---|---|---|
| | day | 0 | 9 | 13 | 16 | 20 | 23 |
| Group | Animal No. | Left hind limb volume (mL) | | | | | |
| Control | 101 | 1.29 | 1.44 | 2.08 | 2.37 | 2.43 | 2.68 |
| | 102 | 1.38 | 1.51 | 2.00 | 2.02 | 1.86 | 1.92 |
| | 103 | 1.36 | 1.42 | 1.74 | 2.11 | 2.00 | 2.09 |
| | 104 | 1.35 | 1.59 | 1.88 | 2.47 | 2.26 | 2.18 |
| | 105 | 1.44 | 1.81 | 2.49 | 2.62 | 2.73 | 3.00 |
| | Mean | 1.36 | 1.55 | 2.04 | 2.32 | 2.26 | 2.37 |
| | S.D. | 0.05 | 0.16 | 0.28 | 0.25 | 0.35 | 0.45 |
| Indomethacin 0.3 mg/kg/day | 201 | 1.39 | 1.47 | 1.78 | 1.74 | 1.71 | 1.69 |
| | 202 | 1.24 | 1.41 | 1.59 | 1.62 | 1.60 | 1.65 |
| | 203 | 1.37 | 1.74 | 2.17 | 2.19 | 2.09 | 1.98 |
| | 204 | 1.30 | 1.79 | 1.98 | 1.95 | 2.06 | 2.20 |
| | 205 | 1.29 | 1.74 | 1.99 | 1.91 | 2.12 | 2.18 |
| | Mean | 1.32 | 1.63 | 1.90 | 1.88 | 1.92 | 1.94 |
| | S.D. | 0.06 | 0.18 | 0.22 | 0.22 | 0.24 | 0.26 |
| Indomethacin 3 mg/kg/day | 301 | 1.17 | 1.36 | 1.39 | 1.36 | 1.32 | 1.33 |
| | 302 | 1.40 | 1.49 | 1.68 | 1.60 | 1.60 | 1.56 |
| | 303 | 1.37 | 1.71 | 1.68 | 1.58 | 1.61 | 1.61 |
| | 304 | 1.32 | 1.49 | 1.69 | 1.62 | 1.51 | 1.58 |
| | 305 | 1.38 | 1.55 | 1.69 | 1.60 | 1.59 | 1.62 |
| | Mean | 1.33 | 1.52 | 1.63 | 1.55 | 1.52 | 1.54 |
| | S.D. | 0.09 | 0.12 | 0.13 | 0.11 | 0.12 | 0.12 |
| GSSSG 100 mg/kg/day | 401 | 1.21 | 1.29 | 1.45 | 1.41 | 1.72 | 1.67 |
| | 402 | 1.39 | 1.52 | 2.35 | 2.64 | 2.82 | 3.22 |
| | 403 | 1.29 | 1.58 | 2.08 | 2.28 | 2.58 | 2.77 |
| | 404 | 1.39 | 1.51 | 2.06 | 2.64 | 2.53 | 2.69 |
| | 405 | 1.29 | 1.58 | 2.00 | 2.35 | 2.31 | 2.34 |
| | Mean | 1.31 | 1.49 | 1.99 | 2.26 | 2.39 | 2.54 |
| | S.D. | 0.07 | 0.12 | 0.33 | 0.50 | 0.42 | 0.58 |
| Indomethacin 0.3 mg/kg/day + GSSSG 100 mg/kg/day | 501 | 1.24 | 1.27 | 1.49 | 1.40 | 1.50 | 1.48 |
| | 502 | 1.25 | 1.37 | 1.68 | 1.65 | 1.76 | 1.85 |
| | 503 | 1.35 | 1.45 | 1.69 | 1.72 | 1.83 | 1.83 |
| | 504 | 1.34 | 1.54 | 1.87 | 1.94 | 1.95 | 1.86 |
| | 505 | 1.33 | 1.75 | 1.67 | 1.65 | 1.60 | 1.63 |
| | Mean | 1.30 | 1.48 | 1.68 | 1.67 | 1.73 | 1.73 |
| | S.D. | 0.06 | 0.18 | 0.13 | 0.19 | 0.18 | 0.17 |
| Indomethacin 3 mg/kg/day + GSSSG 100 mg/kg/day | 601 | 1.35 | 1.43 | 1.48 | 1.45 | 1.44 | 1.49 |
| | 602 | 1.35 | 1.52 | 1.60 | 1.60 | 1.58 | 1.58 |
| | 603 | 1.19 | 1.51 | 1.51 | 1.44 | 1.38 | 1.41 |
| | 604 | 1.34 | 1.52 | 1.53 | 1.48 | 1.49 | 1.49 |
| | 605 | 1.43 | 1.85 | 1.75 | 1.69 | 1.72 | 1.75 |
| | Mean | 1.33 | 1.56 | 1.57 | 1.53 | 1.52 | 1.54 |
| | S.D. | 0.09 | 0.16 | 0.11 | 0.11 | 0.13 | 0.13 |

## Claims

1. A preventive or therapeutic agent for an inflammatory disease and/or a pain disorder, comprising a non-steroidal anti-inflammatory drug or a pharmaceutically acceptable salt thereof, which is administered in combination with glutathione trisulfide or a pharmaceutically acceptable salt thereof.

2. A preventive or therapeutic agent for an inflammatory disease and/or a pain disorder, comprising glutathione trisulfide or a pharmaceutically acceptable salt thereof, which is administered in combination with a non-steroidal anti-inflammatory drug or a pharmaceutically acceptable salt thereof.

3. The preventive or therapeutic agent according to claim 1 or 2,
wherein glutathione trisulfide or the pharmaceutically acceptable salt thereof and the non-steroidal anti-inflammatory drug or the pharmaceutically acceptable salt thereof are administered simultaneously or separately.

4. A preventive or therapeutic agent for an inflammatory disease and/or a pain disorder, comprising glutathione trisulfide or a pharmaceutically acceptable salt thereof; and a non-steroidal anti-inflammatory drug or a pharmaceutically acceptable salt thereof.

5. The preventive or therapeutic agent according to any one of claims 1 to 4, wherein glutathione trisulfide or the pharmaceutically acceptable salt thereof contains at least one selected from the group consisting of glutathione trisulfide, an amino acid salt of glutathione trisulfide, and an alkali metal salt of glutathione trisulfide.

6. The preventive or therapeutic agent according to any one of claims 1 to 4,
wherein glutathione trisulfide or the pharmaceutically acceptable salt thereof contains at least one selected from the group consisting of glutathione trisulfide, an arginine salt of glutathione trisulfide, and a sodium salt of glutathione trisulfide.

7. The preventive or therapeutic agent according to any one of claims 1 to 6,
wherein the non-steroidal anti-inflammatory drug or a pharmaceutically acceptable salt thereof contains at least one selected from the group consisting of a salicylic acid-based non-steroidal anti-inflammatory drug, a propionic acid-based non-steroidal anti-inflammatory drug, and an acetic acid-based non-steroidal anti-inflammatory drug.
